# NEW EUROPEAN PATENT SPECIFICATION

(11) **EP 0 701 443 B2**
(45) Date of publication and mention of the opposition decision: **22.11.2000**
(45) Mention of the grant of the patent: 21.01.1998
(21) Application number: 93918584.9
(22) Date of filing: 03.08.1993
(51) Int. Cl.: A61K 31/445

(54) **TERFENADINE METABOLITES AND THEIR OPTICALLY PURE ISOMERS FOR TREATING ALLERGIC DISORDERS**
Terfanadin Metaboliten und deren optisch reine Isomere zur Behandlung von allergischen Krankheiten
METABOLITES DE TERFENADINE ET LEURS ISOMERES OPTIQUEMENT PURS UTILISES DANS LE TRAITEMENT DES AFFECTIONS ALLERGIQUES

(30) Priority: 03.08.1992 US 924156; 03.08.1992 US 924182
(43) Date of publication of application: 20.03.1996
(60) Divisional application: 97104837.6
(73) Proprietor: SEPRACOR, INC., Marlborough, MA 01752 (US)
(72) Inventor: YOUNG, James, W., Palo Alto, CA 94304 (US); GRAY, Nancy, M., Marlborough, MA 01752 (US); WOOSLEY, Raymond, L., Washington, DC 20016 (US); CHEN, Yiwang, Silver Spring, MD 20906 (US)
(74) Representative: Jump, Timothy John Simon
(86) International application number: PCT/US93/07260
(87) International publication number: WO 94/03170

(56) References cited:
- WO-A-93/23047
- FR-A- 2 453 854
- US-A- 880 801
- US-A- 922 890
- US-A- 4 254 129
- US-A- 4 619 934
- US-A- 4 829 064
- US-A- 4 996 061
- PHARM.RES. vol. 7, no. SUPP , September 1990 page S222 K.Y.CHAN ET AL. 'LACK OF INTERCONVERSION OF THE ENANTIOMERS OF TERFENADINE IN VIVO...'
- J.CHROMATOGRAPHY vol. 570, no. 1 , 18 September 1991 pages 139 - 148 J.E.COUTANT ET AL. 'DETERMINATION OF TERFENADINE AND TERFENADINE ACID METABOLITE...'
- J.CHROMATOGRAPHY vol. 571, no. 1-2 , 1991 pages 291 - 297 K.Y.CHAN ET AL. 'DIRECT ENANTIOMERIC SEPARATION...'
- CHIRALITY vol. 3, no. 6 , 1991 pages 467 - 470 K.ZAMANI ET AL. 'ENANTIOMERIC ANALYSIS OF TERFENADINE IN RAT PLASMA BY HPLC'
- YAKURI TO CHIRYO vol. 16, no. 6 , 1988 pages 2465 - 2480 K.TASAKA ET AL. 'ANTIALLERGIC EFFECTS OF TERFENADINE...'
- ARZNEIM.-FORSCH./DRUG RES. vol. 32(II), no. 9A , 1982 pages 1185 - 1190 D.A.GARTEIZ ET AL. 'PHARMACOKINETICS AND BIOTRANSFORMATION STUDIES OF TERFENADINE IN MAN'
- JAMA vol. 269, no. 12 , 24 March 1993 pages 1532 - 1536 R.L.WOOSLEY 'MECHANISM OF THE CARDIOTOXIC ACTIONS OF TERFENADINE' & pages 1550 - 1552
- J.PHARM.BIOMED.ANAL. vol. 9 , 1991 pages 929 - 933 T.-M.CHEN ET AL. 'DETERMINATION OF THE METABOLITES OF TERFENADINE...'
- CLIN.PHARMACOL.THER. vol. 52, no. 3 , September 1992 pages 231 - 238 P.K.HONIG ET AL. 'CHANGES IN THE PHARMACOKINETICS...'
- ANN.ALLERGY vol. 56, no. 6 , June 1986 pages 464 - 469 K.TASAKA ET AL. 'INTRACELLULAR CALCIUM...'
- IMMUNOPHARMACOL.IMMUNOTOXICOL. vol. 9, no. 2-3 , 1987 pages 259 - 279 M.AKAGI ET AL. 'ANTIALLERGIC EFFECTS OF TERFENADINE...'
- PHARMACEUTISCH WEEKBLAD vol. 125, no. 2 , 1 June 1990 pages 552 - 554 E.J.ARIENS 'RACEMISCHE THERAPEUTICA PROBLEEMMIDDELEN'
- CHIRALITY vol. 2 , 1990 pages 129 - 133 B.TESTA ET AL. 'RACEMATES VERSUS ENANTIOMERS IN DRUG DEVELOPMENT: DOGMATISM OR PRAGMATISM?'
- EUR.J.CLIN.PHARMACOL. vol. 41, no. 2 , 1991 pages 89 - 93 E.J.ARIENS 'RACEMIC THERAPEUTICS - ETHICAL AND REGULATORY ASPECTS'
- SCHWEIZ.MED.WOCHENSCHR. vol. 120, no. 5 , 3 February 1990 pages 131 - 134 E.J.ARIENS 'STEREOSELECTIVITY IN PHARMACODYNAMICS AND PHARMACOKINETICS'

## Description

This invention relates to novel pharmaceutical compositions containing 4-[1-hydroxy-4-(4-hydroxydiphenylmethyl-1-piperidinyl)butyl]-α,α-dimethylbenzeneacetic acid or a pharmaceutically acceptable salt thereof, optionally, in the form of an optically pure derivative. These compositions possess antihistaminic activity and are useful in treating allergic rhinitis. These compositions can be used in combination with non-steroidal anti-inflammatory agents or other non-narcotic analgesics. The aforementioned combinations may optionally include one or more other active components including a decongestant, cough suppressant/antitussive, or expectorant.

Also disclosed are methods for treating the above-described condition in a human while avoiding the adverse effects that are associated with the administration of other α-aryl-4-substituted piperidonoalkanol derivatives, such as terfenadine, by administering the aforementioned pharmaceutical compositions containing 4-[1-hydroxy-4-(4-hydroxydiphenylmethyl-1-piperidinyl)butyl]-α,α- dimethylbenzeneacetic acid, or a pharmaceutically acceptable salt thereof to said human.

The active compound of these compositions, 4-[hydroxy-4-(4-hydroxydiphenylmethyl-1-piperidine)butyl)-α,α-dimethylbenzeneacetic acid is a metabolic derivative of terfenadine. This compound is described in Garteiz et al., Arzneimittel-Forschung/Drug Research, 32: 1185-1190 (1982). Chemically, the optical isomers of the compound are R-(+)-4-[1-hydroxy-4-(4-hydroxydiphenylmethyl-1-piperidinyl)butyl]-α,α-dimethylbenzeneacetic acid, and S-(-)-4-[1-hydroxy-4-(4-hydroxydiphenylmethyl-1-piperidinyl)butyl]-α,α-dimethylbenzeneacetic acid.

### 1.1. Steric Relationship and Drug Action

Many organic compounds exist in optically active forms, i.e., they have the ability to rotate the plane of plane-polarized light. In describing an optically active compound, the prefixes D and L or R and S are used to denote the absolute configuration of the molecule about its chiral center(s). The prefixes d and l or (+) and (-) are employed to designate the sign of rotation of plane-polarized light by the compound, with (-) or l meaning that the compound is levorotatory. A compound prefixed with (+) or d is dextrorotatory. For a given chemical structure, these compounds, called stereoisomers, are identical except that they are mirror images of one another. A specific stereoisomer may also be referred to as an enantiomer, and a mixture of such isomers is often called an enantiomeric or racemic mixture.

Stereochemical purity can be of importance in the field of pharmaceuticals, where 12 of the 20 most prescribed drugs exhibit chirality. A case in point is provided by the L-form of the β-adrenergic blocking agent, propranolol, which is known to be 100 times more potent than the D-enantiomer.

Furthermore, optical purity can be important since certain isomers may actually be deleterious rather than simply inert. For example, it has been suggested that the D-enantiomer of thalidomide was a safe and effective sedative when prescribed for the control of morning sickness during pregnancy, while the corresponding L-enantiomer has been believed to be a potent teratogen.

The enantiomers of 4-[1-hydroxy-4-(4-hydroxydiphenylmethyl-1-piperidinyl)butyl]-α,α-dimethylbenzeneacetic acid are disclosed in Zamani et al., Chirality 3: 467-470 (1991). This reference states that the (R)-enantiomer of an orally administered racemic terfenadine was preferentially oxidized in rats to form a carboxylic acid metabolite enriched in the (R)-enantiomer. The enantiomers of 4-[1-hydroxy-4-(4-hydroxydiphenylmethyl-1-piperidinyl)butyl]-α,α-dimethylbenzeneacetic acid are also disclosed in Chan et al., J. Chromatog. 571: 291-297 (1991). This reference states that terfenadine does not undergo any stereoselective isomeric interconversion in man.

Terfenadine is an antagonist of the H-1 histamine receptor protein. Histamine receptor proteins occur in two well-identified forms in tissues as H-1 and H-2 receptors. The H-1 receptors are those that mediate the response antagonized by conventional antihistamines. H-1 receptors are present in the guinea pig ileum, the skin of Rhesus monkeys, and the bronchial smooth muscle of guinea pig. Terfenadine antagonizes the effect of histamine in the guinea pig isolated ileum, suppresses histamine-induced healing in the skin of Rhesus monkeys, and protects against histamine induced lethality in the guinea pig.

Through H-2 receptor-mediated responses, histamine stimulates gastric acid secretion in the guinea pig and the chronotropic effect in isolated guinea pig atria. Terfenadine has no effect on histamine-induced gastric acid secretion, nor does it alter the chronotropic effect of histamine on atria. Thus, terfenadine has no apparent effect on the H-2 histamine receptor. See Cheng et al., Drug Development Research, 2: 181-196 (1982).

Terfenadine is well absorbed but is extensively metabolized. See Okerholm et al., Biopharmaceutics and Drug Distribution, 2: 185-190 (1981). Two main metabolites have been identified and it has been suggested that one of the metabolites, 4-[1-hydroxy-4-(hydroxydiphenylmethyl-1-piperidinyl)butyl]-α,α-dimethylbenzeneacetic acid, may show antihistaminic activity, in vitro, but no actual data have been published. See Garteiz et al., Arzneimittel-Forschung/Drug Research, 32: 1185-1190 (1982). Also, FR-A-2453854 discloses a large number of substituted piperadine derivatives, including the aforementioned acid metabolite, and suggests that these compounds are useful as antihistamines. However, no substantiating data is provided.

On the basis of its antihistaminic activity, researchers evaluated the effect of terfenadine in the treatment of allergic rhinitis. Clinical trials of efficacy indicated that terfenadine is slightly less effective than chlorpheniramine, another H-1 antagonist. See Connell, Pharmacotherapy, 5: 201-208 (1985).

It has also been suggested that terfenadine would be useful for the treatment of asthma. In guinea pigs, the increase in airway resistance caused by LTD₄ (leukotriene D₄) was suppressed by terfenadine. See Akagi et al., Ovo Yakuri, 35: 361-371 (1988).

Terfenadine may also be useful for the treatment of motion sickness and vertigo. Some antihistamines have been found to be effective for the prophylaxis and treatment of motion sickness. See Wood, Drugs, 17: 471-479 (1979). Some antihistamines have also proven useful for treating vestibular disturbances, such as Meniere's disease, and in other types of vertigo. See Cohen et al., Archives of Neurology, 27: 129-135 (1972).

In addition, terfenadine may be useful in the treatment of diabetic retinopathy and other small vessel disorders associated with diabetes mellitus. In tests on rats with streptozocin-induced diabetes, treatment by antihistamines prevented the activation of retinal histamine receptors which have been implicated in the development of diabetic retinopathy. The use of antihistamines to treat retinopathy and small vessel disorders associated with diabetes mellitus is disclosed in U.S. Patent No. 5,019,591.

It has also been suggested that terfenadine, in combination with non-steroidal anti-inflammatory agents or other non-narcotic analgesics, would be useful for the treatment of cough, cold, cold-like and/or flu symptoms and the discomfort, pain, headache, fever, and general malaise associated therewith. The use of pharmaceutical compositions containing terfenadine and non-narcotic analgesics or non-steroidal anti-inflammatory agents such as aspirin, acetaminophen, and ibuprofen are described in U.S. Patent Nos. 4,783,465 and 4,829,064. These compositions for the treatment of the above-described symptoms may optionally include one or more other active components including a decongestant (such as pseudoephedrine), a cough suppressant/antitussive (such as dextromethorphan) or an expectorant (such as guaifenesin).

Many antihistamines cause somewhat similar adverse effects. These adverse effects include but are not limited to sedation, gastrointestinal distress, dry mouth, and constipation or diarrhea. Terfenadine has been found to cause relatively less sedation, gastrointestinal distress, dry mouth, and constipation or diarrhea, as compared with other antihistamines.

However, the administration of terfenadine to a human has been found to cause other adverse effects. These adverse effects include but are not limited to cardiac arrhythmias, including ventricular tachyarrhythmias, torsades de pointes, and ventricular fibrillation. Recently, clinical practitioners have noted an increase in the occurrence of these cardiac arrhythmias upon coadministration of terfenadine with other drugs such as ketoconazole and erythromycin or upon overdose of terfenadine. See Brian P. Monahan et. al. in JAMA, 5th Dec 1990, Vol. 264, No. 21, p-p 2788-90 and Sandra Knowles in the Canadian Journal of Hospital Pharmacy - Vol. 45, No. 1, 1st February 1992, p.33.

Thus, it would be particularly desirable to find a compound with the advantages of terfenadine which would not have the aforementioned disadvantages.

It has now been discovered that 4-[1-hydroxy-4-(4-hydroxydiphenylmethyl-1-piperidinyl)butyl]-α,α-dimethylbenzeneacetic acid, and its optically pure isomers (hereinafter referred to as "the metabolic derivative of terfenadine" and "optically pure isomers of the metabolic derivative of terfenadine") are useful in treating allergic rhinitis without inducing the cardiac arrhythmias associated with terfenadine.

It has also been discovered that the metabolic derivative of terfenadine and its optically pure isomers can be used in combination with non-steroidal anti-inflammatory agents or other non-narcotic analgesics for treating this condition. Pharmaceutical compositions of the invention, containing (1) the metabolic derivative of terfenadine or its optically pure isomers and (2) non-narcotic analgesics or non-steroidal anti-inflammatory agents such as aspirin, acetaminophen or ibuprofen, may optionally include one or more other active components including a decongestant (such as pseudoephedrine), a cough suppressant/antitussive (such as dextromethorphan) or an expectorant (such as guaifenesin).

Accordingly, and in a first aspect, the present invention provides the use of a pharmaceutical composition comprising a compound of formula I: 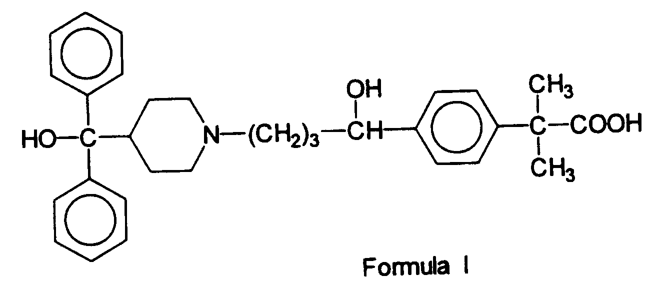 or a pharmaceutically acceptable salt thereof, for the preparation of a medicament for use in a treatment of allergic rhinitis in which the induction of cardiac arrhythmia is avoided, said treatment comprising administering a therapeutically effective amount of a compound of formula I to a human patient whose hepatic function is not impaired. The compound of formula I is the metabolic derivative of terfenadine and it can exist as an optically pure isomer, as aforesaid.

Prior to the present invention, those skilled in the are would have expected the compound of formula I to have induced a form of cardiac arrhythmia known as Torsades de Pointes (as reported in Brian P. Monahan et. al., in JAMA, 5th Dec 1990, Vol. 264, No. 21, p-p 2788-90 and Sandra Knowles in The Canadian Journal of Hospital Pharmacy, Vol. 45, No. 1, 1 Feb 1992, p.33), since this potentially lethal arrythmia was considered to be a "class effect" among non-sedating anti-histamines, in the sense that the arrhythmogenicity was considered to be coupled to the anti-histaminic potency of such compounds. Accordingly, the fact that the compositions, in accordance with the present invention, do not induce any such cardiac arrhythmias is a new, highly useful and surprising technical effect, which enables the inventive compositions to be administered to individuals susceptible to cardiac arrhythmia, and in potentially larger doses than those non-sedating anti-histamines, such as terfenadine, in common use at the present time.

In preferred embodiments, the treatment of allergic rhinitis comprises the administration of the compound of formula I, in an amount of 1-500mg/day and, preferably, in an amount of 20-200mg/day.

In preferred embodiments, the inventive composition further comprises a pharmaceutically acceptable carrier or excipient.

The composition can further comprise a therapeutically effective amount of a non-steroidal anti-inflammatory agent or a non-narcotic analgesic, such as acetylsalicylic acid, acetaminophen, ibuprofen, ketoprofen, or naproxen, or a pharmaceutically acceptable salt thereof. Alternatively, or additionally, a composition in accordance with the present invention can further comprise a therapeutically effective amount of a decongestant, such as pseudoephedrine, or a pharmaceutically acceptable salt thereof.

In a preferred embodiment, the inventive composition comprises from 20mmg to 200mg of the compound of formula I and from 25mg to 600mg of an anti-inflammatory agent or an analgesic. When the inventive composition comprises a therapeutically effective amount of a decongestant, it, preferably, comprises from 20mg to 200mg of a compound of formula I and from 5mg to 150mg of the decongestant.

In preferred embodiments of the present invention, the compound of formula I is in the form of a single optical isomer and the inventive composition is substantially free of the other such isomer. In such an embodiment, the compound of formula I, preferably, is R-(+)-4-[1-hydroxy-4-(4-hydroxydiphenylmethyl-1-piperidinyl)butyl]-α,α-dimethylbenzeneacetic acid, or a pharmaceutically acceptable salt thereof, and the composition is substantially free of the S stereoisomer or, is S-(-)-4-[hydroxy-4-(4-hydroxydiphenylmethyl-1-piperidinyl)butyl]-α,α-dimethylbenzeneacetic acid, or pharmaceutically acceptable salts thereof, and the composition is substantially free of the R stereoisomer.

Preferably, the compound of formula I comprises 90% or more of the selected R- or S-stereoisomer.

In a most preferred embodiment the compound of formula I is terfenadine carboxylate.

Terfenadine has antihistaminic activity and provides therapy and a reduction of symptoms for a variety of conditions and disorders related to allergic disorders, diabetes mellitus and other conditions; however, this drug, while offering the expectation of efficacy, can cause adverse effects including cardiac arrhythmia. Utilizing the metabolic derivative of terfenadine or a substantially optically pure isomer thereof results in clearer dose-related definitions of efficacy, diminished adverse effects, and accordingly, an improved therapeutic index. It is, therefore, more desirable to use the metabolic derivative of terfenadine or an optically pure isomer thereof, than to use terfenadine itself.

The term "adverse effects" includes, but is not limited to cardiac arrhythmias, sedation, gastrointestinal distress, dry mouth, constipation, and diarrhea. The term "cardiac arrhythmias" includes, but is not limited to ventricular tachyarrhythmias, torsades de pointes, and ventricular fibrillation.

The term "substantially free of the S stereoisomer" as used herein means that the metabolic derivative of terfenadine in a composition contains at least 90% by weight of the R isomer of the metabolic derivative of terfenadine, and 10% by weight or less of the S derivative. In a preferred embodiment, the term "substantially free of the S stereoisomer" means that the metabolic derivative of terfenadine in a composition contains at least 99% by weight of the R isomer of the metabolic derivative of terfenadine, and 1% or less of the S isomer. In another preferred embodiment, the term "substantially free of the S stereoisomer" as used herein means that the metabolic derivative of terfenadine in a composition contains greater than 99% by weight of the R isomer of the metabolic derivative of terfenadine and less than 1% by weight of the S derivative. The term "substantially optically pure R isomers of the metabolic derivatives of terfenadine" and "optically pure R isomers of the metabolic derivatives of terfenadine" are also encompassed by the above-described definitions.

The term "substantially free of the R stereoisomer" as used herein means that the composition contains at least 90% by weight of the S isomer of the metabolic derivative of terfenadine, and 10% by weight or less of the R derivative. In a preferred embodiment, the term "substantially free of the R stereoisomer" means that the composition contains at least 99% by weight of the S isomer of the metabolic derivative of terfenadine, and 1% or less of the R isomer. In another preferred embodiment, the term "substantially free of the R stereoisomer" as used herein means that the composition contains greater than 99% by weight of the S isomer of the metabolic derivative of terfenadine and less than 1% by weight of the R derivative. These percentages are based upon the total amount of metabolic derivatives of terfenadine in the composition. The terms "substantially optically pure S isomers of the metabolic derivatives of terfenadine" and "optically pure S isomers of the metabolic derivatives of terfenadine" are also encompassed by the above-described definitions.

The phrase "therapeutically effective amount" means that amount of one or more of the compounds of the invention which provides a therapeutic benefit in the treatment of allergic rhinitis. The symptoms associated with this allergic disorder include, but are not limited to, sneezing, rhinorrhea, lacrimation, and dermal irritation.

The separation of the optically pure isomers of the metabolic derivative of terfenadine may be effected by resolution of a racemic mixture of enantiomers of the metabolic derivative of terfenadine using conventional means such as an optically active resolving acid. Furthermore, the optically pure isomers of the metabolic derivative of terfenadine can be prepared from the racemic mixture by enzymatic biocatalytic resolution. See, for example, United States Patent Nos. 5,057,427 and 5,077,217, the disclosures of which are incorporated by reference.

The magnitude of a prophylactic or therapeutic dose of the metabolic derivative of terfenadine, or an optically pure isomer thereof, in the acute or chronic management of disease will vary with the severity of the condition to be treated and the route of administration. The dose, and perhaps the dose frequency, will also vary according to the age, body weight, and response of the individual patient. In general, the total daily dose range, for the condition described herein, is from about 0.01 mg to about 500 mg administered in single or divided doses orally, topically, transdermally, or locally by aerosol. For example, a preferred oral daily dose range should be from about 1 mg to about 500 mg, while most preferably an oral daily dose range should be between about 20 mg and about 200 mg. It is further recommended that children, patients aged over 65 years, and those with impaired renal function initially receive low doses, and that they then be titrated based on individual response(s) or blood level(s). It may be necessary to use dosages outside these ranges in some cases as will be apparent to those skilled in the art. Further, it is noted that the clinician or treating physician will know how and when to interrupt, adjust, or terminate therapy in conjunction with individual patient response.

Any suitable route of administration may be employed for providing the patient with an effective dosage of the inventive composition. For example, oral, rectal, parenteral, transdermal, subcutaneous, intramuscular, and like forms of administration may be employed. Dosage forms include tablets, troches, dispersions, suspensions, solutions, capsules, patches, and the like.

The pharmaceutical compositions of the present invention comprise the metabolic derivative of terfenadine, or an optically pure isomer thereof as active ingredient, or a pharmaceutically acceptable salt thereof, and may also contain a pharmaceutically acceptable carrier, and optionally, other therapeutic ingredients.

The term "pharmaceutically acceptable salts" includes within its ambit salts prepared from pharmaceutically acceptable non-toxic acids or bases including inorganic acids or bases or organic acids or bases. Examples of such inorganic acids are hydrochloric, hydrobromic, hydroiodic, sulfuric, and phosphoric. Appropriate organic acids may be selected, for example, from aliphatic, aromatic, carboxylic and sulfonic classes of organic acids, examples of which are formic, acetic, propionic, succinic, glycolic, glucoronic, maleic, furoic, glutamic, benzoic, anthranilic, salicylic, phenylacetic, mandelic, embonic (pamoic), methanesulfonic, ethanesulfonic, pantothenic, benzenesulfonic, stearic, sulfanilic, algenic, and galacturonic. Examples of such inorganic bases include metallic salts made from aluminum, calcium, lithium, magnesium, potassium, sodium, and zinc. Appropriate organic bases may be selected, for example, from N,N'-dibenzylethylenediamine, chloroprocaine, choline, diethanolamine, ethylenediamine, meglumaine (N-methylglucamine), lysine and procaine.

The compositions of the present invention include compositions such as suspensions, solutions and elixirs; aerosols, or carriers such as starches, sugars, microcrystalline cellulose, diluents, granulating agents, lubricants, binders, disintegrating agents, and the like, in the case of oral solid preparations (such as powders, capsules, and tablets), with the oral solid preparations being preferred over the oral liquid preparations. The most preferred oral solid preparations are tablets.

Because of their ease of administration, tablets and capsules represent the most advantageous oral dosage unit form, in which case solid pharmaceutical carriers are employed. If desired, tablets may be coated by standard aqueous or nonaqueous techniques.

In addition to the common dosage forms set out above, the compounds of the present invention may also be administered by controlled release means and/or delivery devices such as those described in U.S. Patent Nos. 3,845,770; 3,916,899; 3,536,809; 3,598,123; and 4,008,719.

Pharmaceutical compositions of the present invention suitable for oral administration may be presented as discrete units such as capsules, cachets, or tablets, or aerosol sprays, each containing a predetermined amount of the active ingredient, as a powder or granules, or as a solution or a suspension in an aqueous liquid, a non-aqueous liquid, an oil-in-water emulsion, or a water-in-oil liquid emulsion. Such compositions may be prepared by any of the methods of pharmacy, but all methods include the step of bringing into association the active ingredient with the carrier which constitutes one or more necessary ingredients. In general, the compositions are prepared by uniformly and intimately admixing the active ingredient with liquid carriers or finely divided solid carriers or both, and then, if necessary, shaping the product into the desired presentation.

For example, a tablet may be prepared by compression or molding, optionally, with one or more accessory ingredients. Compressed tablets may be prepared by compressing in a suitable machine the active ingredient in a free-flowing form such as powder or granules, optionally mixed with a binder, lubricant, inert diluent, surface active or dispersing agent. Molded tablets may be made by molding, in a suitable machine, a mixture of the powdered compound moistened with an inert liquid diluent. Desirably, each tablet contains from about 10 mg to about 150 mg of the active ingredient, and each cachet or capsule contains from about 10 mg to about 150 mg of the metabolic derivative of terfenadine. Most preferably, the tablet, cachet or capsule contains either one of three dosages, 30 mg, 60 mg or 90 mg of the active ingredient.

In a second aspect, the present invention provides a pharmaceutical composition in the form of an oral solid preparation comprising a unit dosage of 60 mg of a compound of formula I: 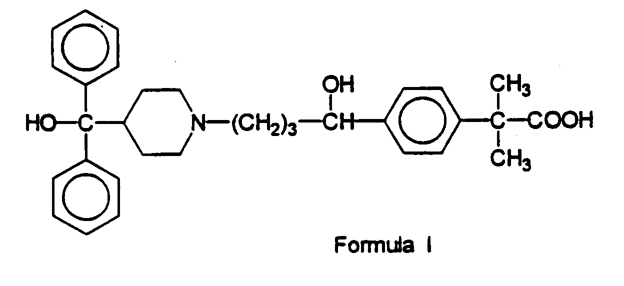 or a pharmaceutically acceptable salt thereof, and a pharmaceutically acceptable carrier or excipient, for use in a treatment of allergic rhinitis in which the induction of cardiac arrhythmia is avoided.

The invention is further defined by reference to the following examples describing in detail the preparation of the compound and the compositions of the present invention, as well as their utility. It will be apparent to those skilled in the art that many modifications, both to materials and methods, may be practiced which are within the scope of this invention.

### EXAMPLES

### Example 1

### A. Preparation of methyl R-4-[1-hydroxy-4-(4-hydroxydiphenylmethyl-1-piperidinyl)butyl]-α,α-dimethylbenzeneacetate.

4-(α-hydroxy-α-phenylbenzyl)piperidine (4.3 gm) was combined with methyl p-(4-chloro-1-oxobutyl)-α,α-dimethylbenzeneacetate (4.5 gm), potassium bicarbonate (2.9 gm), potassium iodide (ca. 50 mg), and methyl isobutyl ketone (50 ml) and heated to reflux for 48 hours. Additional 4-(α-hydroxy-α-phenylbenzyl)piperidine (1.1 gm) was added, and heating was continued for an additional 48 hours. Upon cooling the mixture to room temperature, water was added and the pH of the solution was adjusted to ca. 12 by addition of aqueous sodium hydroxide. The mixture was extracted with ethyl acetate. The ethyl acetate solution was washed with saturated aqueous sodium bicarbonate and brine and dried over sodium sulfate. The ethyl acetate was removed on a rotary evaporator and the residue was treated with 25% ethyl acetate in hexane. The resulting precipitate was filtered and air dried to give methyl 4-[1-oxo-4-(4-hydroxydiphenylmethyl-1-piperidinyl)butyl]-α,α-dimethylbenzeneacetate. This intermediate precipitate (2.4 gm) was combined with tetrahydrofuran (10 ml) and (+)-β-chlorodiisopinocamphenylborane (4.5 gm) and stirred for 48 hours. Methanol (10 ml) and sodium bicarbonate (1.5 gm) were added to the reaction solution, and the mixture was stirred for 12 hours. The mixture was diluted with ethyl acetate (200 ml) and washed with saturated aqueous sodium bicarbonate to give methyl R-4-[1-hydroxy-4-(4-hydroxydiphenylmethyl-1-piperidinyl)butyl]-α,α-dimethylbenzeneacetate.

### B. R-(+)-4-[1-hydroxy-4-(4-hydroxydiphenylmethyl-1-piperidinyl)butyl]-α,α-dimethylbenzeneacetic acid [R-(+)-terfenadine carboxylate].

Methyl R-4-[1-hydroxy-4-(4-hydroxydiphenylmethyl-1-piperidinyl)butyl]-α,α-dimethylbenzeneacetate (1.2 gm) was combined with potassium hydroxide (0.4 gm) and ethanol (5 ml), and the mixture was heated to reflux for 7 hours. The ethanol was removed on a rotary evaporator and the residue was dissolved in water (2 ml). The aqueous solution was acidifed with glacial acetic acid to provide a solid which was recrystallized from 1:1 methanol/ethyl acetate to give R-4-[1-hydroxy-4-(4-hydroxydiphenylmethyl-1-piperidinyl)butyl]-α,α-dimethylbenzeneacetic acid (R-terfenadine carboxylate) (mp=213-215°C).

### C. Preparation of methyl S-4-[1-hydroxy-4-(4-hydroxydiphenylmethyl-1-piperidinyl)butyl)-α,α-dimethylbenzeneacetate.

4-(α-hydroxy-α-phenylbenzyl)piperidine (4.3 gm) was combined with methyl p-(4-chloro-1-oxobutyl)-α,α-dimethylbenzene-acetate (4.5 gm), potassium bicarbonate (2.9 gm), potassium iodide (ca. 50 mg), and methyl isobutyl ketone (50 ml) and heated to reflux for 48 hours. Additional 4-(α-hydroxy-α-phenylbenzyl)piperidine (1.1 gm) was added, and heating was continued for an additional 48 hours. Upon cooling the mixture to room temperature, water was added and the pH of the solution was adjusted to ca. 12 by addition of aqueous sodium hydroxide. The mixture was extracted with ethyl acetate. The ethyl acetate solution was washed with saturated aqueous sodium bicarbonate and brine and dried over sodium sulfate. The ethyl acetate was removed on a rotary evaporator and the residue was treated with 25% ethyl acetate in hexane. The resulting precipitate was filtered and air dried to give methyl 4-[1-oxo-4-(4-hydroxydiphenyl-methyl-1-piperidinyl)butyl]-α,α-dimethylbenzeneacetate. This intermediate precipitate (2.4 gm) was combined with tetrahydrofuran (10 ml) and (-)-β-chlorodiisopinocamphenylborane (4.5 gm) and stirred for 48 hours. Methanol (10 ml) and sodium bicarbonate (1.5 gm) were added to the reaction solution and the mixture was stirred for 12 hours. The mixture was diluted with ethyl acetate (200 ml) and washed with saturated aqueous sodium bicarbonate to give methyl S-4-[1-hydroxy-4-(4-hydroxydiphenylmethyl-1-piperidinyl)butyl]-α,α-dimethylbenzeneacetate. If the aforementioned intermediate precipitate was to be reacted with racemic β-chlorodiisopinocamphenylborane, then a racemic mixture of methyl 4-[1-hydroxy-4-(4-hydroxydiphenylmethyl-1-piperidinyl)butyl]-α,α-dimethylbenzeneacetate would be produced.

### D. S-(-)-4-[1-hydroxy-4-(4-hydroxydiphenylmethyl-1-piperidinyl)butyl]-α,α-dimethylbenzeneacetic acid [S-(-)-terfenadine carboxylate].

Methyl S-4-[1-hydroxy-4-(4-hydroxydiphenylmethyl-1-piperidinyl)butyl]-α,α-dimethylbenzeneacetate (1.2 gm) was combined with potassium hydroxide (0.4 gm) and ethanol (5 ml) and the mixture was heated to reflux for 7 hours. The ethanol was removed on a rotary evaporator and the residue was dissolved in water (2 ml). The aqueous solution was acidifed with glacial acetic acid to provide a solid which was recrystallized from 1:1 methanol/ethyl acetate to give S-(-)-4-[1-hydroxy-4-(4-hydroxydiphenylmethyl-1-piperidinyl)butyl]-α,α-dimethylbenzeneacetic acid (S-terfenadine carboxylate) (mp=215-218°C).

### EXAMPLE 2

Activities of the compounds of the invention at the histamine H₁-receptor were assessed using the [³H]pyrilamine binding assay as described in Chang et al., J. Neurochem. 32: 1653-1663 (1979). Briefly, membranes from bovine cerebellum were incubated with [³H]pyrilamine and varying concentrations of test compound. The reactions were carried out in 50 mM sodium phosphate buffer (pH 7.5) at 25°C for 30 minutes. The reaction was terminated by rapid vacuum filtration onto glass fiber filters. Radioactivity trapped on the filters was determined and compared to control values to ascertain the interaction of the test compound with the H₁-receptor. Results were as follows:

| Compound | Percent Inhibition (at various concentrations) | | |
|---|---|---|---|
| | 10⁻⁹M | 10⁻⁷M | 10⁻⁵M |
| R,S-terfenadine | 11.0 | 28.7 | 86.9 |
| R-(+)-terfenadine | 11.4 | 19.4 | 90.3 |
| R-(+)-terfenadine carboxylate | 12.4 | 45.2 | 87.3 |
| S-(-)-terfenadine | 3.2 | 24.4 | 92.8 |
| S-(-)-terfenadine carboxylate | 8.1 | 54.1 | 88.7 |

### Example 3

Single ventricular myocytes were obtained from isolated cat hearts by conventional techniques. The rod-shaped single cells were maintained in a HEPES buffer and they were "patch clamped" using suction pipettes. A Patch-Clamp L/M-PEC 7 amplifier was used to record current tracings and the recording electrodes were filled with a solution of potassium aspartate. Voltage clamp pulses and data acquisition were controlled by a Sperry PC/IT Computer running P Clamp software. A minimum of 4 cells were studied at each test concentration of the following drugs: racemic terfenadine, racemic terfenadine carboxylate, and quinidine (as a reference compound). Results were as follows:

| | Conc (µM) | Block of the delayed rectifier potassium current (%) |
|---|---|---|
| Terfenadine | 0.01 | 12 ± 9.3 |
| | 0.10 | 39.5 ± 9.8 |
| | 1.00 | 92.6 (92.5; 92.8) |
| Terfenadine carboxylate | 0.01 | 0 ± 0 |
| | 0.10 | 0 ± 0 |
| | 1.00 | 0 ± 0 |

These results show that terfenadine carboxylate, surprisingly, is not liable to cause cardiac arrhythmia, at dose levels at which there is a distinct risk of such a side effect being caused by terfenadine itself.

### Example 4

### Oral Formulation - Capsules:

| Formula | Quantity per capsule in mg. | | |
|---|---|---|---|
| | A | B | C |
| Active ingredient (S)Terfenadine carboxylate | 30.0 | 60.0 | 90.0 |
| Starch 1500 | 69.0 | 39.0 | 9.0 |
| Magnesium Stearate BP | 1.0 | 1.0 | 1.0 |
| Compression Weight | 100.0 | 100. | 100.0 |

The active ingredient, which can instead be (R)terfenadine carboxylate or racemic terfenadine carboxylate, is sieved and blended with the excipients.

The mixture is filled into suitably sized two-piece hard gelatin capsules using suitable machinery. Other doses may be prepared by altering the fill weight and if necessary, changing the capsule size to suit.

### Example 5

### Oral Formulation - Tablets:

| Formula | Quantity per Tablet in mg. | | |
|---|---|---|---|
| | A | B | C |
| Active ingredient, (R)Terfenadine Carboxylate | 30.0 | 60.0 | 90.0 |
| Lactose BP | 123.5 | 93.5 | 63.5 |
| Starch BP | 30.0 | 30.0 | 30.0 |
| Pregelatinized Maize Starch BP | 15.0 | 15.0 | 15.0 |
| Magnesium Stearate | 1.5 | 1.5 | 1.5 |
| Compression Weight | 200.0 | 200.0 | 200.0 |

The active ingredient, which can instead be (S)terfenadine carboxylate or racemic terfenadine carboxylate, is sieved through a suitable sieve and blended with the lactose until a uniform blend is formed. Suitable volumes of water are added and the powders are granulated. After drying, the granules are then screened and blended with the magnesium stearate. The resulting granules are then compressed into tablets of desired shape. Tablets of other strengths may be prepared by altering the ratio of active ingredient to the excipient(s) of the compression weight.

## Claims

1. Use of a composition comprising a compound of formula I: 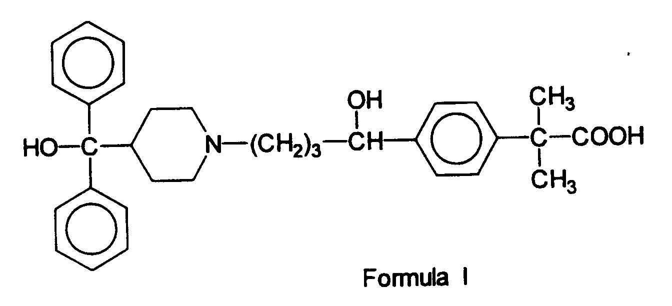 or a pharmaceutically acceptable salt thereof, for the preparation of a medicament for use in a treatment of allergic rhinitis in which the induction of cardiac arrhythmia is avoided, said treatment comprising administering a therapeutically effective amount of a compound of formula I to a human patient whose hepatic function is not impaired.

2. A use as claimed in claim 1, wherein the anti-histaminic treatment comprises the administration of a compound of formula I in an amount of 1-500mg/day and, preferably, in an amount of 20-200mg/day.

3. A use as claimed in claim 1 or claim 2, wherein the composition further comprises a pharmaceutically acceptable carrier or excipient.

4. A use as claimed in any of the preceding claims, wherein the composition further comprises a therapeutically effective amount of a non-steroidal anti-inflammatory agent or a non-narcotic analgesic.

5. A use as claimed in claim 4, wherein the composition comprises from 20mg to 200mg of a compound of formula I and from 25mg to 600mg of the anti-inflammatory agent or analgesic.

6. A use as claimed in any of the preceding claims, wherein the composition further comprises a therapeutically effective amount of a decongestant.

7. A use as claimed in claim 6, wherein the composition comprises from 20mg to 200mg of a compound of formula I and from 5mg to 150mg of a decongestant.

8. A use as claimed in any of the preceding claims, wherein the compound of formula I is in the form of a single optical isomer and the composition is substantially free of the other such isomer.

9. A use as claimed in claim 8, wherein the compound of formula I comprises 90% or more, by weight, R-(+) stereoisomer.

10. A use as claimed in claim 8, wherein the compound of formula I comprises 90% or more, by weight, S-(-) stereoisomer.

11. A use as claimed in any of the proceeding claims, wherein the compound of formula I is terfenadine carboxylate.

12. A pharmaceutical composition in the form of an oral solid preparation comprising a unit dosage of 60mg of a compound of formula I: 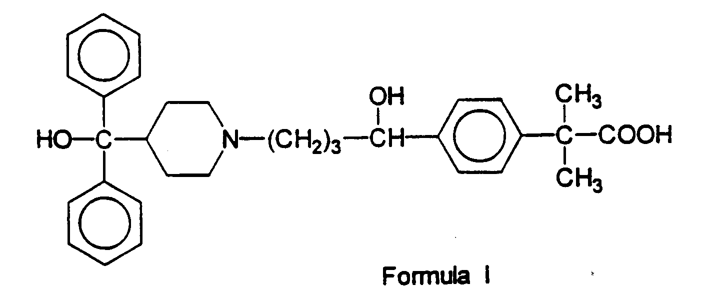 or a pharmaceutically acceptable salt thereof, and a pharmaceutically acceptable carrier or excipient, for use in a treatment of allergic rhinitis in which the induction of cardiac arrhythmia is avoided.

13. A pharmaceutical composition, as claimed in claim 12 further comprising a therapeutically effective amount of a non-steroidal anti-inflammatory agent or a non-narcotic analgesic.

14. A pharmaceutical composition, as claimed in claim 13, comprising from 25mg to 600mg of the anti-inflammatory agent or analgesic.

15. A pharmaceutical composition, as claimed in any of claims 12-14, further comprising a therapeutically effective amount of a decongestant.

16. A pharmaceutical composition, as claimed in claim 15, comprising from 5mg to 150mg of a decongestant.

17. A pharmaceutical composition, as claimed in any of claims 12-16, wherein the compound of formula I is in the form of a single optical isomer and the composition is substantially free of the other such isomer.

18. A pharmaceutical composition, as claimed in claim 17, wherein the compound of formula I comprises 90% or more, by weight, R-(+) stereoisomer.

19. A pharmaceutical composition, as claimed in claim 17, wherein the compound of formula I comprises 90% or more, by weight, S-(-) stereoisomer.

20. A pharmaceutical composition, as claimed in any of claims 12-19, wherein the compound of formula I is terfenadine carboxylate.

## Patentansprüche

1. Verwendung einer Zusammensetzung mit einer Verbindung gemäß folgender Formel I: 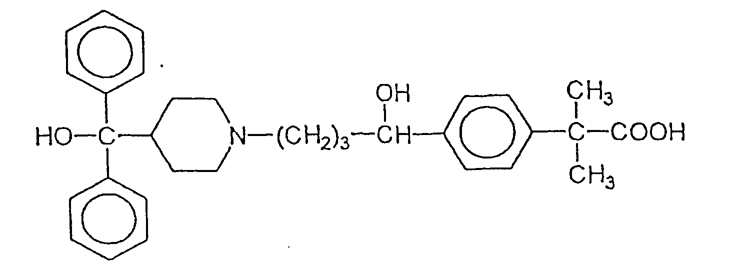 oder eines pharmazeutisch annehmbaren Salzes davon zur Zubereitung eines Medikamentes zur Anwendung bei der Behandlung von allergischer Rhinitis, bei der die Herbeiführung einer Herzarrhythmie vermieden wird, wobei die Behandlung eine Verabreichung einer therapeutisch wirksamen Menge einer Verbindung gemäß Formel I einem menschlichen Patienten umfaßt, dessen hepatische Funktion nicht beeinträchtigt ist.

2. Verwendung nach Anspruch 1, bei der die antihistamine Behandlung die Verabreichung einer Verbindung gemäß Formel I mit einer Menge von 1-500mg/Tag und vorzugsweise von 20-200 mg/Tag umfaßt.

3. Verwendung nach Anspruch 1 oder 2, bei der die Zusammensetzung ferner einen pharmazeutisch annehmbaren Träger oder Excipienten aufweist.

4. Verwendung nach einem der vorhergehenden Ansprüche, bei der die Zusammensetzung ferner eine therapeutisch wirksame Menge eines nichtsteroidalen entzündungshemmenden Mittels oder eines nichtnarkotischen schmerzstillenden Mittels aufweist.

5. Verwendung nach Anspruch 4, bei der die Zusammensetzung zwischen 20 und 200 mg der Verbindung gemäß Formel I und zwischen 25 und 600 mg des entzündungshemmenden Mittels oder des schmerzstillenden Mittels aufweist.

6. Verwendung nach einem der vorhergehenden Ansprüche, bei der die Zusammensetzung ferner eine therapeutisch wirksame Menge eines abschwellenden Mittels aufweist.

7. Verwendung nach Anspruch 6, bei der die Zusammensetzung zwischen 20 und 200 mg der Verbindung gemäß Formel I und zwischen 5 und 150 mg des abschwellenden Mittels aufweist.

8. Verwendung nach einem der vorhergehenden Ansprüche, bei der die Verbindung gemäß Formel I in Form eines einzelnen optischen Isomers vorliegt und die Zusammensetzung im wesentlichen frei von anderen solchen Isomeren ist.

9. Verwendung nach Anspruch 8, bei der die Verbindung gemäß Formel I 90 Gewichts% oder mehr eines R-(+) Stereoisomers aufweist.

10. Verwendung nach Anspruch 8, bei der die Verbindung gemäß Formel I 90 Gewichts% oder mehr eines S-(-) Stereoisomers aufweist.

11. Verwendung nach einem der vorhergehenden Ansprüche, bei der die Verbindung gemäß Formel I Terfenadincarboxylat ist.

12. Pharmazeutische Zusammensetzung in Form einer festen oralen Zubereitung mit einer Dosierung je Einheit von 60 mg einer Verbindung gemäß folgender Formel I: 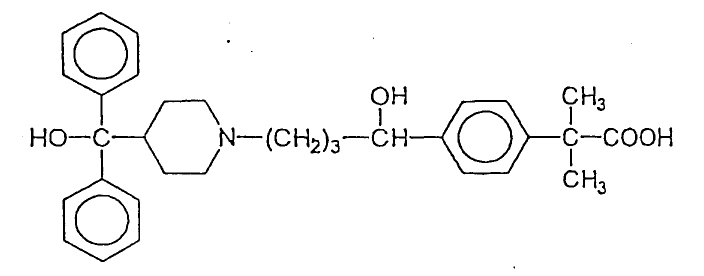 oder eines pharmazeutisch annehmbaren Salzes davon und eines pharmazeutisch annehmbaren Trägers oder Excipienten, zur Anwendung bei der Behandlung von allergischer Rhinitis, bei der die Herbeiführung einer Herzarrhythmie vermieden wird.

13. Pharmazeutische Zusammensetzung nach Anspruch 12, die ferner eine therapeutisch wirksame Menge eines nichtzsteroidalen entzündungshemmenden Mittels oder eines nichtnarkotischen schmerzstillenden Mittels aufweist.

14. Pharmazeutische Zusammensetzung nach Anspruch 13, die zwischen 25 und 600 mg des entzündungshemmenden Mittels oder des schmerzstillenden Mittels aufweist.

15. Pharmazeutische Zusammensetzung nach einem der Ansprüche 12 bis 14, die ferner eine therapeutisch wirksame Menge eines abschwellenden Mittels aufweist.

16. Pharmazeutische Zusammensetzung nach Anspruch 15, die zwischen 5 und 150 mg des abschwellenden Mittels aufweist.

17. Pharmazeutische Zusammensetzung nach einem der Ansprüche 12 bis 16, bei der die Verbindung gemäß Formel I in Form eines einzelnen optischen Isomers vorliegt und die Zusammensetzung im wesentlichen frei von anderen solchen Isomeren ist.

18. Pharmazeutische Zusammensetzung nach Anspruch 17, bei der die Verbindung gemäß Formel I 90 Gewichts% oder mehr eines R-(+) Stereoisomers aufweist.

19. Pharmazeutische Zusammensetzung nach Anspruch 17, bei der die Verbindung gemäß Formel I 90 Gewichts% oder mehr eines S-(-) Stereoisomers aufweist.

20. Pharmazeutische Zusammensetzung nach einem der Ansprüche 12 bis 19, bei der die Verbindung gemäß Formel I Terfenadincarboxylat ist.

## Revendications

1. Utilisation d'une composition comprenant un composé de formule I: 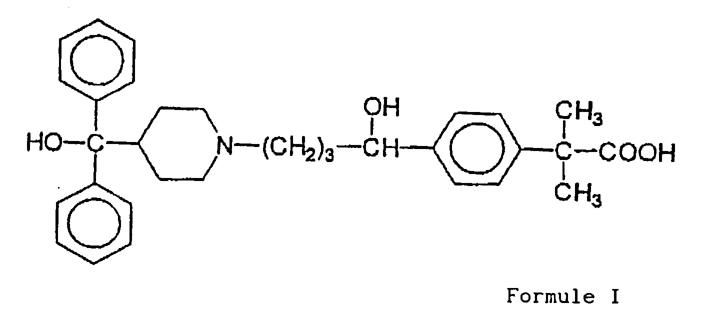 ou un sel pharmaceutiquement acceptable de celui-ci, pour la fabrication d'un médicament destiné au traitement de la rhinite allergique, dans lequel est évitée l'induction d'arythmie cardiaque, ledit traitement comprenant l'administration d'une quantité thérapeutiquement efficace d'un composé de formule I à un patient humain dont la fonction hépatique n'est pas altérée.

2. Utilisation selon la revendication 1, dans laquelle le traitement antihistaminique comprend l'administration d'un composé de formule I en une quantité de 1-500 mg/jour et, de préférence, en une quantité de 20-200 mg/jour.

3. Utilisation selon la revendication 1 ou 2, dans laquelle la composition comprend en outre un véhicule ou excipient pharmaceutiquement acceptable.

4. Utilisation selon l'une quelconque des revendications précédentes, dans laquelle la composition comprend en outre une quantité thérapeutiquement efficace d'un agent anti-inflammatoire non stéroïdien ou d'un analgésique non narcotique.

5. Utilisation selon la revendication 4, dans laquelle la composition comprend de 20 mg à 200 mg d'un composé de formule I et de 25 mg à 600 mg de l'agent anti-inflammatoire ou de l'analgésique.

6. Utilisation selon l'une quelconque des revendications précédentes, dans laquelle la composition comprend en outre une quantité thérapeutiquement efficace d'un décongestionnant.

7. Utilisation selon la revendication 6, dans laquelle la composition comprend de 20 mg à 200 mg d'un composé de formule I et de 5 mg à 150 mg d'un décongestionnant.

8. Utilisation selon l'une quelconque des revendications précédentes, dans laquelle le composé de formule I est sous la forme d'un seul isomère optique et la composition est pratiquement exempte de l'autre isomère.

9. Utilisation selon la revendication 8, dans laquelle le composé de formule I comprend 90 % ou plus, en poids, de stéréoisomère R-(+).

10. Utilisation selon la revendication 8, dans laquelle le composé de formule I comprend 90 % ou plus, en poids, de stéréoisomère

11. Utilisation selon l'une quelconque des revendications précédentes, dans laquelle le composé de formule I est le carboxylate de terfénadine.

12. Composition pharmaceutique sous la forme d'une préparation solide orale comprenant un dosage unitaire de 60 mg du composé de formule I : 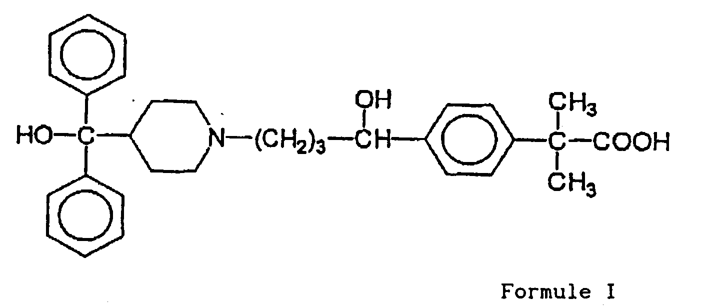 ou d'un sel pharmaceutiquement acceptable de celui-ci, et un véhicule ou excipient pharmaceutiquement acceptable, pour utilisation dans un traitement de la rhinite allergique, dans lequel est évitée l'induction d'arythmie cardiaque.

13. Composition pharmaceutique selon la revendication 12, comprenant en outre une quantité thérapeutiquement efficace d'un agent anti-inflammatoire non stéroïdien ou d'un analgésique non narcotique.

14. Composition pharmaceutique selon la revendication 13, comprenant de 25 mg à 600 mg de l'agent anti-inflammatoire ou de l'analgésique.

15. Composition pharmaceutique selon l'une quelconque des revendications 12 à 14, comprenant en outre une quantité thérapeutiquement efficace d'un décongestionnant.

16. Composition pharmaceutique selon la revendication 15, comprenant de 5 mg à 150 mg d'un décongestionnant.

17. Composition pharmaceutique selon l'une quelconque des revendications 12 à 16, dans laquelle le composé de formule I est sous la forme d'un seul isomère optique et la composition est pratiquement exempte de l'autre isomère.

18. Composition pharmaceutique selon la revendication 17, dans laquelle le composé de formule I comprend 90 % ou plus, en poids, de stéréoisomère R-(+).

19. Composition pharmaceutique selon la revendication 17, dans laquelle le composé de formule I comprend 90 % ou plus, en poids, de stéréoisomère S-(-).

20. Composition pharmaceutique selon l'une quelconque des revendications 12 à 19, dans laquelle le composé de formule I est le carboxylate de terfénadine.
